# EUROPÄISCHE PATENTSCHRIFT

(11) **EP 0 736 293 B1**
(45) Veröffentlichungstag und Bekanntmachung des Hinweises auf die Patenterteilung: **05.01.2000**
(21) Anmeldenummer: 96104643.0
(22) Anmeldetag: 23.03.1996
(51) Int. Cl.: A61F 2/42

(54) **Fingergelenk**
Finger joint
Articulation de doigt

(30) Priorität: 06.04.1995 DE 19512854
(43) Veröffentlichungstag der Anmeldung: 09.10.1996
(73) Patentinhaber: ESKA Implants GmbH & Co., 23556 Lübeck (DE)
(72) Erfinder: Grundei, Hans, Dr., 23558 Lübeck (DE); Rudigier, Jürgen, Prof. Dr., 77652 Offenburg (DE); Weber, Christian, Dr., 01844 Hohwald (DE)
(74) Vertreter: Fuchs Mehler Weiss & Fritzsche

(56) Entgegenhaltungen:
- DE-A- 2 522 377
- DE-A- 3 008 292
- DE-B- 2 146 253
- FR-A- 2 651 119
- US-A- 3 638 243
- US-A- 4 304 011
- US-A- 5 147 386

## Beschreibung

Die Erfindung betrifft ein Fingergelenk. Es kann implantiert werden nach Resektion sowohl eines Fingergrundgelenks zwischen den Handknochen und den Fingerknöcheln, als auch eines Fingergelenkes zwischen den Fingerknöcheln. Ein Fingergelenk gemäß den Merkmalen des Oberbegriffs des Anspruchs 1 ist aus der FR-A-26 51 119 bekannt.

In den meisten Fällen werden Fingergelenke reseziert, wenn diese rheumatisch sind und dem Patienten große Schmerzen zufügen. Bislang wurde an die Stelle des natürlichen Gelenkes ein sogenanntes Swanson-Gelenk gesetzt, bei dem es sich allerdings nicht um ein echtes Gelenk, sondern vielmehr um einen Silikonplatzhalter handelt mit zwei in die Röhrenknochen einsetzbaren länglichen Ankern, welche mit einem Grundkörper aus Silikon verbunden sind. Die Flexibilität des Silikon bietet dem Patienten eine gewisse Beweglichkeit des behandelten Fingers. Allerdings wurde bei Anwendung des Swanson-Gelenks beobachtet, daß sich um dieses ''Gelenk'' eine Bindegewebskapsel bildet, welche der Beweglichkeit und der Dauerhaftigkeit des Verbleibs des Swanson-Gelenks in situ abträglich ist.

Vor diesem Hintergrund ist es die Aufgabe der vorliegenden Erfindung, ein Fingergelenk zu schaffen, welches eine hohe Genauigkeit der Nachbildung der natürlichen physiologischen Bewegung eines natürlichen Fingergelenks erreicht. Darüber hinaus sollen die Voraussetzungen für ein für den Patienten möglichst angenehmes Tragen geschaffen werden, um so eine möglichst hohe Akzeptanz zu erzielen.

Die Aufgabe wird gelöst durch ein Fingergelenk mit den Merkmalen des Anspruchs 1. Weitere vorteilhafte Ausgestaltungen ergeben sich aus den Unteransprüchen.

Demgemäß wird also zunächst vorgeschlagen, daß das Fingergelenk einen ersten und einen zweiten hohlen, in die Fingerröhrenknochen bzw. Handröhrenknochen setzbaren Verankerungsstiel (Hohlstiel) aufweist, zwischen denen ein Scharniergelenk angeordnet ist. Vorteilhaft sind die besagten Verankerungsstiele gebildet aus einem offenmaschigen Gitternetzwerk, vorzugsweise aus Metall, durch welches hindurch Knochenbälkchen wachsen können und so für eine dauerhafte Verankerung der Schäfte im Knochen sorgen können.

Der erste Stiel ist mit einem Kugelkäfig des Scharniergelenks verbunden, in welchem eine mit einem Stiel versehene Gelenkkugel gelagert ist, wobei der Stiel den Kugelkäfig durch einen darin vorgesehenen Schlitz durchtritt und mit dem zweiten Hohlstiel in Verbindung steht. Der Schlitz im Kugelkäfig erweitert sich von der Stelle, an der der Stiel den Kugelkäfig in Streckstellung des Gelenks durchtritt, zu der Stelle, an der der Stiel den Kugelkäfig in Beugestellung des Gelenks durchtritt, stetig.

Demnach erlaubt das Kugelgelenk nicht nur die übliche Beugebewegung eines Scharniergelenks von der Streckstellung zur Beugestellung, sondern erhöht das Spiel mit zunehmender Beugestellung des Gelenks, und zwar in der senkrecht auf der Beugungsebene stehenden Ebene. Dies entspricht annähernd dem Bewegungsablauf eines natürlichen Fingergelenks. Die Bedeutung des möglichst genau nachempfundenen physiologischen Bewegungsablaufes liegt vor allem darin, daß die das Gelenk umgebenden Weichteile, wie Bindegewebe und Sehnen nach der Implantation ihre Funktionen in einem gegenüber dem natürlichen Zustand praktisch unveränderten Umfeld ausüben können. Hierdurch wird der Tragekomfort beim Patienten gesteigert und dadurch die Akzeptanz des Implantates gefördert.

Die Verbindung des Scharniergelenks mit den beiden hohlen, im Knochen sitzenden Stielen erfolgt vorzugsweise über konische Klemmverbindungen, die seit langem im Bereich der Implantattechnik bekannt sind.

Gemäß einer bevorzugten Ausführungsform ist der Kugelkäfig selbst eine Kugel. Der besagte Schlitz, durch welchen der Stiel der Gelenkkugel greift, durchsetzt die Wandung des nun kugelförmigen Kugelkäfigs auf einem Abschnitt eines Meridians von einem Pol bis etwa zum Äquator der Kugel. Durch die Ausbildung des Kugelkäfigs in Gestalt einer Kugel ergibt sich ein kompaktes Implantat, welches die problemlose Führung von Sehnen etc. über die Kugeloberfläche im implantierten Zustand des Fingergelenkes ermöglicht. Dies führt zur schonenden Behandlung der Weichteile und Sehnen des Fingers und infolgedessen zu einer hohen Verträglichkeit des Implantates.

Um eine möglichst hohe Verträglichkeit zu erreichen und beispielsweise Metallose zu verhindern, ist gemäß einer vorteilhaften Weiterbildung vorgesehen, daß die innere Gelenkkugel einstückig mit dem besagten Stiel ausgebildet ist und mit einer Polyethylenbeschichtung versehen ist. Vorzugsweise ist die gesamte Gelenkkugel mit Polyethylen überzogen. Darüber hinaus ist vorteilhaft der besagte Stiel im Ansatzbereich an der Gelenkkugel ebenfalls mit dem Polyethylen beschichtet in dem Bereich, in welchem er die Wandung des Kugelkäfigs durchtritt. Somit wird wirksam ein Metall/Metallkontakt vermieden, wenn das Implantat aus Metall gefertigt ist.

Falls es trotz aller Sorgfalt doch einmal zu einem Revisionseingriff kommen sollte, damit die Gelenkkugel ausgetauscht werden kann, ist gemäß einer vorteilhaften Weiterbildung eine Polkappe von mindestens der Größe der Gelenkkugel vorgesehen, die nach Art eines Deckels mit dem Kugelkäfig verschraubbar ist und dessen kugelförmige Außengestaltung komplettiert. Die sehr kompakte Einheit läßt sich also durch ein Aufschrauben der Polkappe vom Kugelkörper auftrennen, woraufhin dann die Gelenkkugel mit dem angeformten Stiel entfernt werden kann. In einfacher Weise kann ein neues Teil aus Gelenkkugel und Stiel in den Kugelkäfig eingesetzt werden und die Polkappe wieder verschlossen werden.

Die Wirkungsweise des erfindungsgemäßen Fingergelenks kann noch erhöht werden. Hierzu sei allerdings vorausgemerkt, daß es auch bei dem natürlichen Fingergelenk nicht nur ausschließlich zu einer Beugebewegung mit der erwähnten Spielvergrößerung kommt. Es findet auch eine Längenstreckung statt. So werden die natürlichen Sehnen bei Beugung des natürlichen Gelenkes mehr unter Zug gesetzt als dies der Fall in der Streckstellung des Fingers ist. Um diese Längenänderungen zu kompensieren ist gemäß einer vorteilhaften Weiterbildung vorgesehen, daß der Stiel an der Gelenkkugel des Fingergelenks in eine Schiebehülse, welche mit dem besagten zweiten Hohlstiel verbindbar ist, quasi teleskopisch greift, derart, daß der Stiel in der Hülse längsverschieblich ist. Hierdurch kann sich der Stiel in der Hülse zur Kompensation der Längenänderungen verschieblich bewegen und so für eine Ausgleichbewegung sorgen.

Vorzugsweise besteht auch die Schiebehülse aus Polyethylen. Sie kann mit dem zweiten hohlen Stiel durch eine übliche konische Klemmverbindung dauerhaft verbunden sein.

Vorzugsweise weist die Schiebehülse an ihrem dem Kugelkäfig zugewandten Ende eine Manschette auf, welche sich im zusammengebauten Zustand des Gelenkes an die Außenseite des Kugelkäfigs anlegt. Hierdurch wird eine zumindest teilweise Abdeckung des Schlitzes durch die Wandung des Kugelkäfigs erzielt. Am besten freilich läßt sich dies erzielen in dem Falle, daß auch der Kugelkäfig selbst außen eine Kugel ist. Dann weist die Manschette der Schiebehülse eine Teilkalottenform auf und kann den Schlitz praktisch vollständig bedecken, so daß der Kugelkäfig im Inneren weitgehend frei bleibt von physiologischen Flüssigkeiten etc., die die guten Lagereigenschaften des Gelenkes über längere Zeit in Mitleidenschaft ziehen könnten.

Die Erfindung wird anhand eines Ausführungsbeispieles gemäß der Zeichnungsfiguren näher erläutert. Hierbei zeigt:
- Fig. 1: sämtliche Teile des Fingergelenkes im nicht montierten Zustand, wobei in der Fig. 1a eine Ansicht auf den Kugelkäfig in Richtung der Pfeile A in Fig. 1 dargestellt ist, und
- Fig. 2: eine Schnittansicht durch das montierte Fingergelenk in Beugestellung.

Nachfolgend bezeichnen die gleichen Bezugszeichen dieselben Teile.

Kernstück des erfindungsgemäßen Fingergelenks 1 ist der Kugelkäfig 5 des Scharniergelenks 4. Vorliegend ist der Kugelkäfig 5 selbst kugelförmig ausgebildet und wird durch die Polkappe 10 in seiner Kugelform komplettiert. Hierzu kann das Gewinde 16 an der Polkappe 10 mit dem im Kugelkäfig 5 vorgesehenen Innengewinde 17 verschraubt werden.

Radial von der Polkappe 10 steht ein Klemmkonus 15 ab, der mit einer konischen Klemmhülse 14 in dem ersten Hohlstiel 2 sicher, aber auch lösbar mittels einer konischen Klemmverbindung verbindbar ist. Vorliegend besteht der Hohlstiel 2 aus einem offenmaschigen Gitternetzwerk, durch welches hindurch Knochenbälkchen der den Schaft 2 nach der Implantation umgebenden Spongiosa einwachsen können zur dauerhaften Fixation des Implantates.

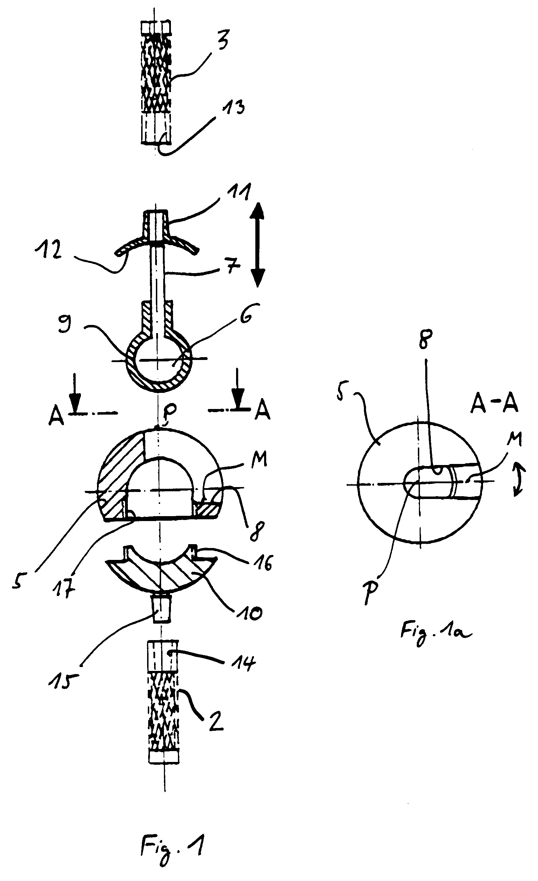

Die Wandung des Kugelkäfigs 5 wird im Bereich von ihrem Pol bis in den Bereich ihres Äquators durchsetzt von einem Führungsschlitz 8. Dieser Führungsschlitz 8 erweitert sich vom Bereich um den Pol P hin zum Bereich des Äquators des Kugelkäfigs 5 stetig, wie deutlich aus Fig. 1a zu erkennen ist. Hierdurch wird dem Stiel 7, der an die Gelenkkugel 6 angeformt ist (Fig. 1) in der Beugestellung des Fingergelenks ein größeres Spiel in Richtung des Doppelpfeiles in Fig. 1a verliehen als dies der Fall ist bei gestrecktem Fingergelenk, wo das Spiel praktisch gleich null ist. Dies entspricht weitgehend dem physiologischen Bewegungsablauf eines natürlichen Fingergelenkes.

Vorliegend ist die Gelenkkugel 6 beschichtet mit einer Polyethylenschicht 9, die auch noch den unteren Bereich des angeformten Stiels 7 mit bedeckt, und zwar so weit, daß kein Kontakt zwischen dem (metallischen) Stiel 7 und dem (metallischen) Kugelkäfig 5 im Bereich des Schlitzes 8 stattfinden kann. Hierdurch wird wirksam einer Metallose vorgebeugt.

Es ist ein zweiter Hohlstiel 3 vorgesehen, der vorliegend genau so ausgebildet ist, wie der erste Hohlstiel 2, also aus einem offenmaschigen Gitternetzwerk besteht und eine konische Klemmhülse 13 aufweist. Mit der konischen Klemmhülse in eine konische Klemmverbindung bringbar ist eine Schiebehülse 11, die zu diesem Zwecke die Außenkonturen eines Klemmkonus aufweist. Im Inneren ist die Schiebehülse 11 allerdings zylindrisch ausgebildet, derart, daß der Stiel 7 in der Schiebehülse 11 längsverschieblich gelagert ist. Hierdurch wird eine quasi teleskopische Bewegung des Stieles in der Schiebehülse 11 auch nach der Implantation des Fingergelenkes in Richtung des Doppelpfeiles in Fig. 1 ermöglicht, wodurch Längenkompensationsbewegungen ausgeführt werden können.

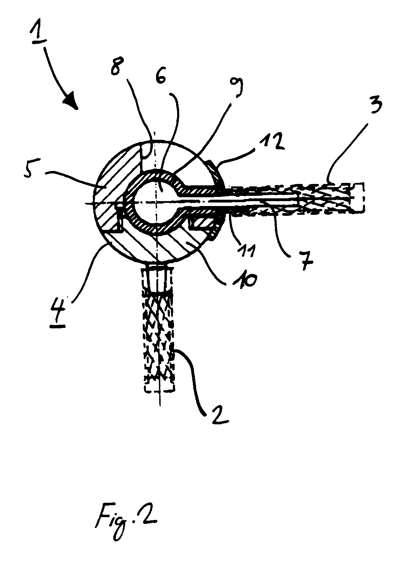

Vorliegend weist die Schiebehülse 11 an ihrem dem hier kugelförmig ausgebildeten Kugelkäfig 5 des Scharniergelenks 4 zugewandten Ende eine Manschette 12 auf, die sich im zusammengebauten Zustand des Fingergelenks (Fig. 2) an die Außenseite des Kugelkäfigs 5 legt. Hier ist die Manschette 12 als Teil einer Kugelkalotte anzusehen. Die Manschette 12 dient dazu, den Schlitz 8, durch welchen der Stiel 7 greift, zumindest teilweise abzudecken, um ein Eindringen von physiologischen Flüssigkeiten wie Blut etc. in das Innere des Kugelkäfigs zu vermeiden. Die Manschette 12 kann in konkreten Ausführungsbeispielen wesentlich größer sein als in den Zeichnungsfiguren dargestellt.

Das dargestellte Ausführungsbeispiel gestattet, wie aus Fig. 2 ersichtlich, eine Beugebewegung von bis zu 90°. Auf dieses Maß ist das erfindungsgemäße Fingergelenk allerdings nicht festgelegt. Durch Verlängerung des Schlitzes ist auch beispielsweise ein Beugebereich von 100° möglich.

Die Montage der Teile aus Fig. 1 erfolgt in anschaulicher Weise dadurch, daß das Gelenkkugelteil mit Stiel 7 in den Kugelkäfig 5 gesetzt wird, wonach der Stiel 7 durch den Schlitz 8 greift. Hiernach wird die Polkappe 10 mit dem übrigen Kugelkäfig 5 verschraubt. Über den Stiel 7 wird die Schiebehülse 11 gestreift, solange bis die Manschette 12 an der Außenwandung des Kugelkäfigs 5 anliegt. Sodann werden die konischen Klemmverbindungen zwischen der Klemmhülse 14 im Hohlstiel 2 und dem Klemmkonus 15 an der Polkappe 10 einerseits und zwischen der konischen Klemmhülse 13 im Hohlstiel 3 und dem konischen Klemmkonus, welcher an der Außenseite der Schiebehülse 11 ausgeformt ist, andererseits hergestellt.

## Patentansprüche

1. Fingergelenk (1) mit einem ersten und einem zweiten, in die Fingerröhrenknochen einsetzbaren Verankerungstiel (2, 3), zwischen denen ein Scharniergelenk (4) in Form eines Kugelgelenks angeordnet ist, bei dem der erste Verankerungsstiel (2) mit einem Kugelkäfig (5) des Scharniergelenks (4) verbunden ist, in welchem eine mit einem Stiel (7) versehene Gelenkkugel (6) gelagert ist, wobei der Stiel (7) den Kugelkäfig (5) durch einen darin vorgesehenen Schlitz (8) hindurchtritt und mit dem zweiten Verankerungsstiel (3) in Verbindung steht dadurch gekennzeichnet, daß die Verankerungsstiele (2,3) hohe ausgebildet sind, und daß sich der Schlitz (8) im Kugelkäfig (5) von der Stelle (P), an der der Stiel (7) den Kugelkäfig (5) in Streckstellung des Gelenks (4) durchtritt, zu der Stelle (M), an der der Stiel (7) den Kugelkäfig (5) in Beugestellung des Gelenks durchtritt, stetig aufweitet.

2. Fingergelenk nach Anspruch 1, bei dem der Kugelkäfig (5) selbst eine Kugel ist und der Schlitz (8) auf einem Teil eines Meridians von einem Pol (P) bis etwa zum Äquator die Wandung des kugelförmigen Kugelkäfigs (5) durchsetzt.

3. Fingergelenk nach Anspruch 1 oder 2, bei dem die Gelenkkugel (6) einstückig mit dem Stiel (7) ausgebildet ist und mit einer Polyethylenbeschichtung versehen ist.

4. Fingergelenk nach einem der Ansprüche 2 bis 3, bei dem eine Polkappe (10) von mindestens der Größe der Gelenkkugel (6, 9) vorgesehen ist, die nach Art eines Deckels mit dem Kugelkäfig (5) verschraubbar ist und dessen kugelförmige Außengestalt komplettiert.

5. Fingergelenk nach einem der Ansprüche 1 bis 4, bei dem der Stiel (7) an der Gelenkkugel (5) in eine Schiebehülse (11), welche mit dem zweiten Hohlstiel (3) verbindbar ist, greift, derart, daß der Stiel (7) in der Hülse (11) längsverschieblich zur Ausführung von Längenkompensationsbewegungen ist.

6. Fingergelenk nach Anspruch 5, bei dem die Schiebehülse (11) aus Polyethylen besteht.

7. Fingergelenk nach Anspruch 5 oder 6, bei dem die Schiebehülse (11) an ihrem dem Kugelkäfig (5) zugewandten Ende eine Manschette (12) aufweist, welche sich im zusammengebauten Zustand des Gelenks an die Außenseite des Kugelkäfigs anlegt.

8. Fingergelenk nach den Ansprüchen 2 und 7, bei dem die Manschette (12) teilkalottenförmig entsprechend dem kugelförmigen Kugelkäfig (5) ausgebildet ist.

## Claims

1. Finger joint (1) having a first and a second anchoring stem (2, 3) which can be inserted in the long bones of the finger, and between which a hinge joint (4) is arranged in the form of a ball joint, in which the first anchoring stem (2) is connected to a ball cage (5) of the hinge joint (4), in which a joint ball (6) provided with a stem (7) is mounted, wherein the stem (7) passes through the ball cage (5) through a slot (8) provided therein and is connected to the second anchoring stem (3), characterised in that the anchoring stems (2, 3) are designed to be hollow, and in that the slot (8) in the ball cage (5) expands continuously from the position (P), at which the stem (7) passes through the ball cage (5) in the extended position of the joint (4), to the position (M), at which the stem (7) passes through the ball cage (5) in the bending position of the joint.

2. Finger joint according to claim 1, in which the ball cage (5) itself is a sphere and the slot (8) penetrates the wall of the spherical ball cage (5) on a part of a meridian from a pole (P) approximately to the equator.

3. Finger joint according to claim 1 or 2, in which the joint ball (6) is designed to be integral with the stem (7) and is prodded with a polyethylene coating.

4. Finger joint according to one of claims 2 to 3, in which a pole cap (10) of at least the size of the joint ball (6, 9) is provided, which can be screwed to the ball cage (5) like a cover and completes its spherical external form.

5. Finger joint according to one of claims 1 to 4, in which the stem (7) on the joint ball (5) extends into a sliding sleeve (11), which can be connected to the second hollow stem (3), such that the stem (7) can be displaced longitudinally in the sleeve (11) to execute longitudinal compensation movements.

6. Finger joint according to claim 5, in which the sliding sleeve (11) consists of polyethylene.

7. Finger joint according to claim 5 or 6, in which the sliding sleeve (11) has at its end facing the bail cage (5) a collar (12) which rests against the outer side of the ball cage in the assembled state of the joint.

8. Finger joint according to claims 2 and 7, in which the collar (12) is designed to be a partial dome shape corresponding to the spherical ball cage (5).

## Revendications

1. Articulation de doigt (1) avec des première et deuxième tiges d'ancrage (2, 3) pouvant être mises en place dans les os longs des doigts, entre lesquelles est agencée une articulation à charnière (4) sous la forme d'une rotule, dans laquelle la première tige d'ancrage (2) est reliée à un logement de rotule (5) de l'articulation à charnière (4) dans lequel vient se loger une rotule (6) munie d'une tige (7), la tige (7) traversant le logement de rotule (5) par une fente (8) prévue dans ce dernier et étant en liaison avec la deuxième tige d'ancrage (3), caractérisée en ce que les tiges d'ancrage (2, 3) ont une configuration creuse et en ce que la fente (8) du logement de rotule (5) s'élargit constamment de l'endroit (P) où la tige (7) traverse le logement de rotule (5) dans la position d'extension de l'articulation (4) jusqu'à l'endroit (M) où la tige (7) traverse le logement de rotule (5) dans la position de flexion de l'articulation.

2. Articulation de doigt selon la revendication 1, dans laquelle le logement de rotule (5) est lui-même une sphère et la fente (8) traverse la paroi du logement de rotule (5) sphérique sur une partie d'un méridien allant d'un pôle (P) à peu près jusqu'à l'équateur.

3. Articulation de doigt selon la revendication 1 ou la revendication 2, dans lequel la rotule (6) est formée d'un seul tenant avec la tige (7) et est munie d'un revêtement en polyéthylène.

4. Articulation de doigt selon l'une des revendications 2 à 3, dans laquelle il est prévu une calotte polaire (10) ayant au moins la taille de la rotule (6, 9) qui peut se visser à la manière d'un couvercle sur le logement de rotule (5) et qui complète la forme sphérique de ce dernier.

5. Articulation de doigt selon l'une des revendications 1 à 4, dans laquelle la tige (7) est en prise au niveau du logement de rotule (5) avec une douille coulissante (11) qui peut être reliée à la deuxième tige creuse (3), de telle sorte que la tige (7) peut se déplacer longitudinalement dans la douille coulissante (11) pour effectuer des mouvements de compensation en longueur.

6. Articulation de doigt selon la revendication 5, dans laquelle la douille coulissante (11) est en polyéthylène.

7. Articulation de doigt selon la revendication 5 ou la revendication 6, dans laquelle la douille coulissante (11) présente à celles de ses extrémités qui est dirigée vers le logement de rotule (5) une manchette (12) qui, dans l'état assemblé de l'articulation, est en contact contre l'extérieur du logement de rotule.

8. Articulation de doigt selon la revendication 2 et la revendication 7, dans laquelle la manchette (12) a la forme d'une partie de calotte correspondant au logement de rotule (5) sphérique.
